(19) Europäisches Patentamt
European Patent Office
Office européen des brevets

(11) Veröffentlichungsnummer: **0 160 908**
**A2**

(12) **EUROPÄISCHE PATENTANMELDUNG**

(21) Anmeldenummer: 85105168.0

(22) Anmeldetag: 27.04.85

(51) Int. Cl.[4]: C 07 D 239/48
C 07 D 239/38, A 01 N 43/54

(30) Priorität: 10.05.84 DE 3417264

(43) Veröffentlichungstag der Anmeldung: 13.11.85 Patentblatt 85/46

(84) Benannte Vertragsstaaten: AT BE CH DE FR GB IT LI NL

(71) Anmelder: BAYER AG
Konzernverwaltung RP Patentabteilung
D-5090 Leverkusen 1 Bayerwerk(DE)

(72) Erfinder: Schwamborn, Michael, Dr.
von-Lohe-Strasse 9
D-5000 Köln 80(DE)

(72) Erfinder: Kühle, Engelbert, Dr.
von-Bodelschwingh-Strasse 42
D-5060 Bergisch-Gladbach 2(DE)

(72) Erfinder: Klauke, Erich, Dr.
Eichendorffweg 8
D-5068 Odenthal(DE)

(72) Erfinder: Eue, Ludwig, Dr.
Paul-Klee-Strasse 36
D-5090 Leverkusen 1(DE)

(72) Erfinder: Schmidt, Robert R., Dr.
Im Waldwinkel 110
D-5060 Bergisch-Gladbach 2(DE)

(72) Erfinder: Santel, Hans-Joachim, Dr.
Gerstenkamp 19
D-5000 Köln 80(DE)

(72) Erfinder: Hänssler, Gerd, Dr.
Heymannstrasse 40
D-5090 Leverkusen 1(DE)

(54) **Neue 2,4-Diamino-6-halogen-5-alkylthio-pyrimidine.**

(57) Die Erfindung betrifft neue, 2,4-Diamino-6-halogen-5-alkylthio-pyrimidine der allgemeinen Formel (I)

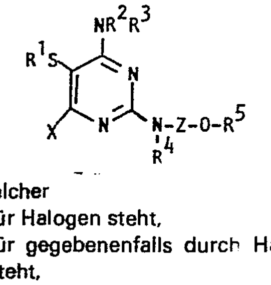

(I)

in welcher
X für Halogen steht,
$R^1$ für gegebenenfalls durch Halogen substituiertes Alkyl steht,
$R^2$ für Wasserstoff oder Alkyl steht,
$R^3$ für Wasserstoff, Alkyl oder Alkoxyalkyl steht,
$R^4$ für Wasserstoff oder Alkyl steht,
Z für eine verzweigte oder unverzweigte Alkylengruppe steht und
$R^5$ für Alkyl steht,

Verfahren zu ihrer Herstellung und ihre Verwendung als Pflanzenschutzmittel, insbesondere als Herbizide und Fungizide.

BAYER AKTIENGESELLSCHAFT 5090 Leverkusen, Bayerwerk
Konzernverwaltung RP
Patentabteilung Bi/Ke-c
Ib

Neue 2,4-Diamino-6-halogen-5-alkylthio-pyrimidine

Die vorliegende Erfindung betrifft neue 2,4-Diamino-6-halogen-5-alkylthio-pyrimidine, Verfahren und neue Zwischenprodukte zu ihrer Herstellung sowie ihre Verwendung als Pflanzenschutzmittel, insbesondere als Herbizide und Fungizide.

Es ist bereits bekannt, daß bestimmte 2,4-Diamino-6-chlor-5-alkylthio-pyrimidine als Herbizide eingesetzt werden können (vgl. EP-A-0 000 681). So kann beispielsweise 2,4-Diamino-6-chlor-5-methylthio-pyrimidin als selektives Herbizid in Getreide, Mais, Reis und Sorghum im Vor- und Nachauflaufverfahren eingesetzt werden; seine Wirkung bei niedrigen Aufwandmengen ist jedoch bei verschiedenen Schadpflanzen nicht befriedigend.

Die vorbekannte Verbindungsgruppe soll auch eine gewisse fungizide Wirksamkeit besitzen (vgl. FR-A-2 119 234), jedoch erweist sich die Wirksamkeit bei einer Reihe von Pilzerkrankungen unter Praxisbedingungen als nicht ausreichend. Insbesondere ist über eine Wirkung dieser Verbindungen gegen Pyricularia oryzae an Reis nichts bekannt.

Es wurden nun neue 2,4-Diamino-6-halogen-5-alkylthio-pyrimidine der allgemeinen Formel (I)

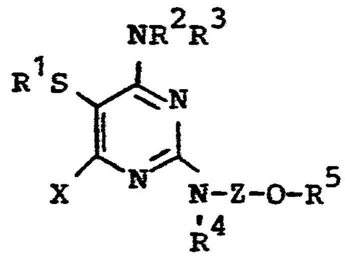

(I)

in welcher

X für Halogen steht,

$R^1$ für gegebenenfalls durch Halogen substituiertes Alkyl steht,

$R^2$ für Wasserstoff oder Alkyl steht,

R3 für Wasserstoff, Alkyl oder Alkoxyalkyl steht,

$R^4$ für Wasserstoff oder Alkyl steht,

Z für eine verzweigte oder unverzweigte Alkylengruppe steht und

$R^5$ für Alkyl steht,

aufgefunden.

Weiterhin wurde gefunden, daß man die Pyrimidinderivate der allgemeinen Formel (I) erhält, wenn man Trihalogenpyrimidine der allgemeinen Formel (II)

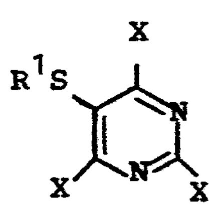

(II)

in welcher

$R^1$ und X die oben angegebene Bedeutung haben,

entweder

(A) zunächst mit einem Alkoxyalkylamin der Formel (III)

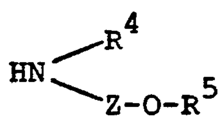

(III)

worin

$R^4$, Z und $R^5$ die oben angegebene Bedeutung haben,

in Gegenwart eines säurebindenden Mittels und gegebenenfalls in Gegenwart eines Verdünnungsmittels zu einem Gemisch der isomeren Pyrimidinderivate der allgemeinen Formeln (IVa) und (IVb)

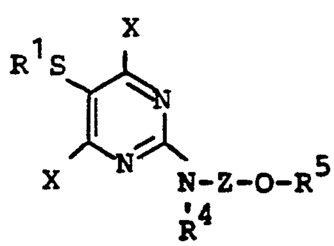

+

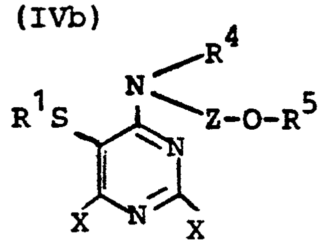

(IVa)

(IVb)

worin

$R^1$, X, $R^4$, Z und $R^5$ die oben angegebene Bedeutung haben,

umsetzt (1. Stufe) und anschließend nach Trennung der beiden Isomeren das Pyrimidinderivat der Formel (IVa) in einem weiteren Reaktionsschritt mit einem Amin der Formel (V)

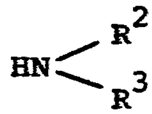

(V)

worin

$R^2$ und $R^3$ die oben angegebene Bedeutung haben,

in Gegenwart eines säurebindenden Mittels und gegebenenfalls in Gegenwart eines Verdünnungsmittels zum Pyrimidinderivat (I) umsetzt (2. Stufe),

oder

(B) zunächst mit einem Amin der Formel (V) - unter den gleichen Reaktionsbedingungen wie bei Verfahren (A) - zu einem Gemisch der isomeren Pyrimidinderivate der allgemeinen Formeln (VIa) und (VIb)

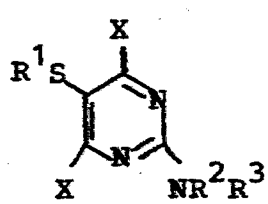

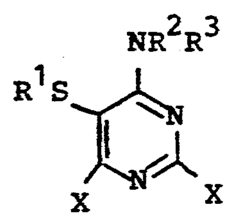

(VIa) (VIb)

worin

$R^1$, X, $R^2$ und $R^3$ die oben angegebene Bedeutung haben,

umsetzt (1. Stufe) und anschließend nach Trennung der beiden Isomeren das Pyrimidinderivat der Formel (VIb) in einem weiteren Reaktionsschritt mit einem Alkoxyalkylamin der Formel (III) - wiederum unter den gleichen Reaktionsbedingungen wie bei Verfahren (A) - zu einem Gemisch der isomeren Pyrimidinderivate der allgemeinen Formeln (I) und (Ia)

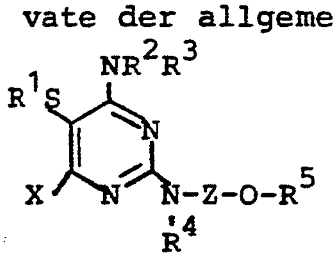

und

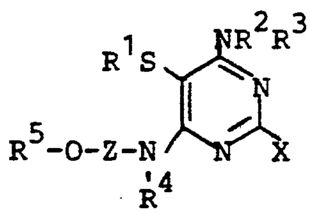

(I) (Ia)

worin

$R^1$, X, $R^2$, $R^3$, $R^4$, Z und $R^5$ die oben angegebene Bedeutung haben,

umsetzt (2. Stufe) und schließlich die gewünschten Isomeren der Formel (I) abtrennt.

Außerdem wurde gefunden, daß die neuen Pyrimidinderivate der allgemeinen Formel (I) starke herbizide Eigenschaften aufweisen.

Die erfindungsgemäßen neuen Pyrimidinderivate der Formel (I) zeichnen sich gegenüber den vorbekannten Pyrimidinen strukturell insbesondere dadurch aus, daß die 2-Aminogruppe eine geradkettige oder verzweigte Alkoxyalkylaminogruppe ist.

Überraschenderweise sind die erfindungsgemäßen Wirkstoffe der Formel (I), bei gleicher Verträglichkeit für Mais und Weizen, deutlich wirksamer als die vorbekannten Pyrimidinderivate. Insbesondere hat sich gezeigt, daß die erfindungsgemäßen Wirkstoffe gegen Panicum, Chenopodium, Poa und Setaria erheblich besser wirksam sind als das vorbekannte 2,4-Diamino-6-chlor-5-methylthiopyrimidin.

Darüberhinaus wurde gefunden, daß die erfindungsgemäßen Pyrimidinderivate bei niedrigen Konzentrationen, die für eine Herbizidanwendung nicht infrage kommen, auch starke fungizide Eigenschaften aufweisen.

Von den erfindungsgemäßen Pyrimidinderivaten der Formel (I) sind bevorzugt diejenigen, in denen

X für Chlor oder Fluor steht,

$R^1$ für gegebenenfalls durch Chlor und/oder Fluor substituiertes Alkyl mit 1 bis 6 C-Atomen steht,

$R^2$, $R^3$ und $R^4$ jeweils unabhängig voneinander für Wasserstoff oder Alkyl mit 1 bis 6 C-Atomen stehen,

$R^3$ zusätzlich für $(C_1-C_6)$-Alkoxy-$(C_1-C_6)$-alkyl steht,

Z für eine verzweigte oder unverzweigte Alkylengruppe mit 2 bis 10 C-Atomen steht und

$R^5$ für Alkyl mit 1 bis 6 C-Atomen steht.

Aus dieser Stoffgruppe sind solche Verbindungen der Formel (I) besonders bevorzugt, in denen

X für Chlor oder Fluor steht,

$R^1$ für gegebenenfalls durch Fluor substituiertes Alkyl mit 1 bis 4 C-Atomen steht,

$R^2$, $R^3$ und $R^4$ jeweils unabhängig voneinander für Wasserstoff oder Alkyl mit 1 bis 4 C-Atomen steht,

$R^3$ zusätzlich für $(C_1-C_4)$-Alkoxy-$(C_1-C_4)$-alkyl steht,

Z für eine verzweigte oder unverzweigte Alkylengruppe mit 2 bis 7 C-Atomen steht und

$R^5$ für Alkyl mit 1 bis 4 C-Atomen steht.

Verwendet man gemäß Verfahren (A) beispielsweise 2,4,6-Trichlor-5-methylthio-pyrimidin und 3-Isopropoxypropylamin als Ausgangsstoffe und setzt das hierbei gebildete 4,6-Dichlor-2-(3-isopropoxypropylamino)-5-methylthio-pyrimidin weiter mit Ammoniak um, so kann der Reaktionsablauf zusammenfassend durch das folgende Formelschema wiedergegeben werden:

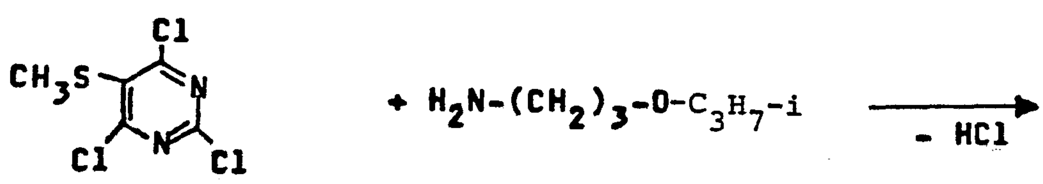

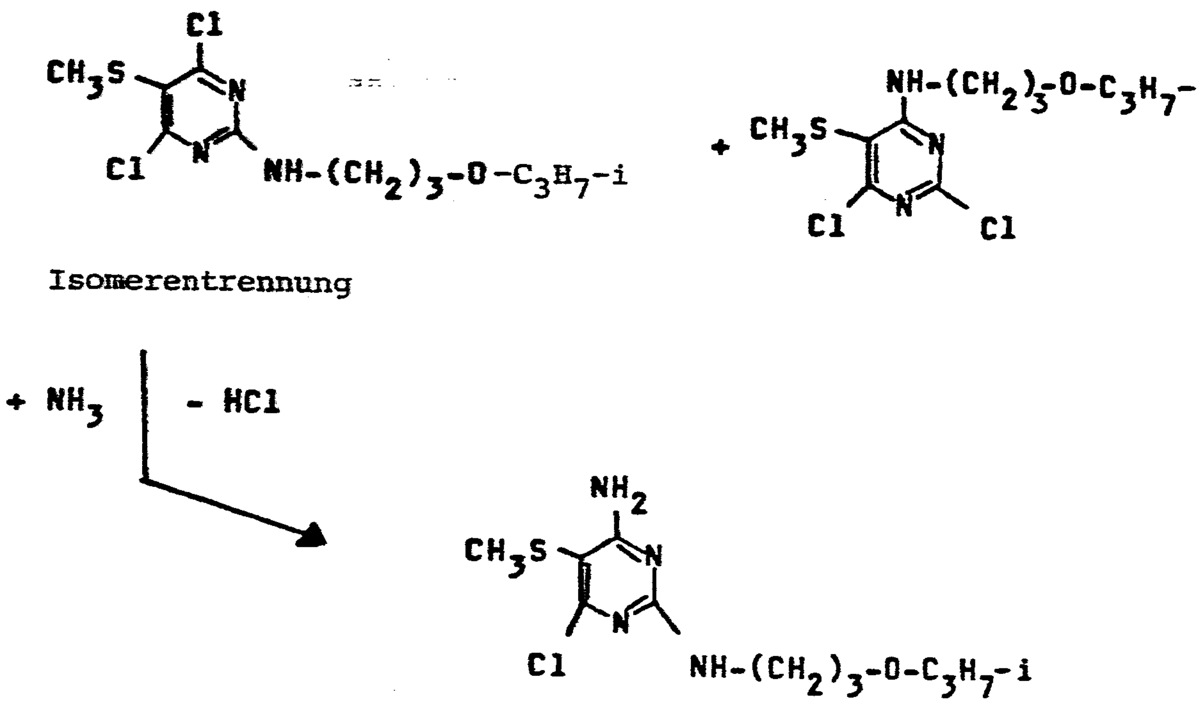


Verwendet man gemäß Verfahren (B) beispielsweise 2,4,6-Trifluor-5-methylthio-pyrimidin und Ammoniak als Ausgangsstoffe und setzt das hierbei gebildete 4-Amino-2,6-difluor-5-methylthio-pyrimidin mit 3-Methoxypropylamin um, so kann der Reaktionsablauf zusammenfassend durch das folgende Formelschema wiedergegeben werden:

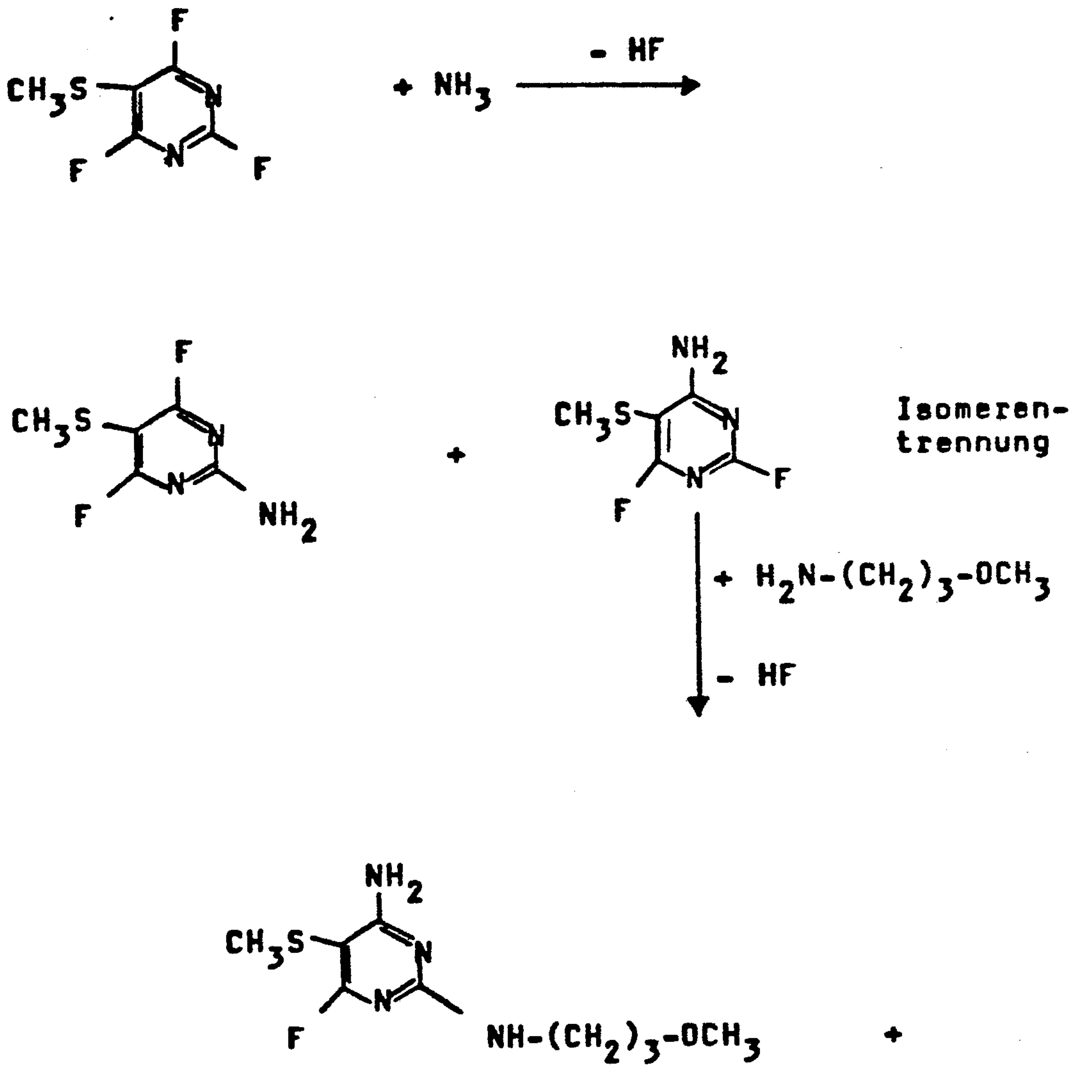


Isolierung durch Isomerentrennung

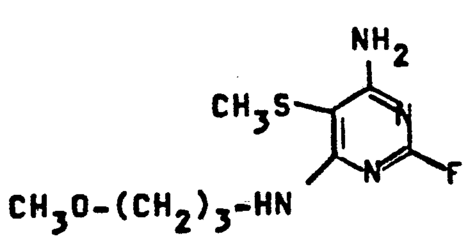

Die als Ausgangsstoffe verwendeten Trihalogenpyrimidine sind durch die Formel (II) allgemein definiert. In dieser Formel stehen $R^1$ und X vorzugsweise bzw. besonders bevorzugt für diejenigen Reste, die schon bei der Beschreibung der erfindungsgemäßen Stoffe der Formel (I) als bevorzugt bzw. besonders bevorzugt für diese Substituenten genannt wurden. Die Trihalogenpyrimidine der Formel (II) sind zum Teil bekannt (X = Cl: FR-PS 1.549.494) bzw. können nach bekannten Verfahren hergestellt werden.

Die weiterhin als Ausgangsstoffe verwendeten Amine sind durch die Formeln (III) und (V) allgemein definiert. In diesen Formeln stehen $R^2$, $R^3$, $R^4$, $R^5$ und Z vorzugsweise bzw. besonders bevorzugt für diejenigen Reste, die schon bei der Beschreibung der erfindungsgemäßen Stoffe der Formel (I) als bevorzugt bzw. besonders bevorzugt für diese Substituenten genannt wurden. Die Amine der Formeln (III) und (V) sind bekannt oder lassen sich nach bekannten Verfahren in analoger Weise wie die bekannten Verbindungen herstellen (vgl. z.B. Houben-Weyl, Methoden der organischen Chemie, Band XI/1, S. 548, S. 561 ff., 4. Auflage 1957).

Als Verdünnungsmittel für das erfindungsgemäße Verfahren kommen organische Lösungsmittel sowie Wasser infrage. Bevorzugte organische Lösungsmittel sind Kohlenwasserstoffe wie Toluol, aliphatische Ketone wie Aceton, Methylethylketon und Diethylketon, cycloaliphatische Ether wie Tetrahydrofuran oder Dioxan. Auch Gemische verschiedener organischer Lösungsmittel und Gemische von mit Wasser mischbaren organischen Lösungsmitteln mit Wasser sind als Verdünnungsmittel geeignet.

Das erfindungsgemäße Verfahren wird unter Verwendung von Säurebindemitteln durchgeführt. Als solche sind Erdalkali- und Alkalimetallhydroxide, wie Calcium-, Natrium- oder Kaliumhydroxid, ferner Ammoniak sowie tertiäre aliphatische Amine wie z.B. Triethylamin, aber auch im Überschuß eingesetztes Amin-Ausgangsprodukt (III) bzw. (V) besonders geeignet.

Die Reaktionstemperaturen können bei dem erfindungsgemäßen Verfahren in einem größeren Bereich variiert werden. Die erste Verfahrensstufe wird im allgemeinen bei Temperaturen von -30 bis +150°C, vorzugsweise von -20 bis +30°C durchgeführt; bei der zweiten Verfahrensstufe arbeitet man im allgemeinen bei 80 bis 150°C, bevorzugt bei 90 bis 130°C.

Die Umsetzung wird im Druckbereich von 1 bis etwa 10 bar durchgeführt.

Bei der Durchführung des erfindungsgemäßen Verfahrens setzt man auf 1 Mol Trihalogenpyrimidin der Formel (II) in der ersten Stufe im allgemeinen 1 bis 1,1 Mol Amin der Formel (III) bzw. (V) und 1 bis 1,2 Mol Säurebinder ein, wobei als Säurebinder das Amin (III) bzw. (V) verwendet werden kann. Bevorzugt wird unter Einsatz stöchiometrischer Molverhältnisse gearbeitet. Für die zweite Verfahrensstufe gilt Entsprechendes.

Die bei beiden Verfahrensstufen anfallenden Isomerengemische können in einfacher Weise nach bekannten Methoden

getrennt werden, insbesondere durch Umkristallisieren (vgl. z.B. FR-A 2 119 231), so daß sich die jeweils gewünschten Isomeren in ausreichend reiner Form isolieren lassen.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe können - insbesondere im Nachauflaufverfahren - zur selektiven Unkrautbekämpfung in monokotylen Kulturen, z.B. in Mais und Getreide, eingesetzt werden. Die neuen Wirkstoffe sind bei gleicher Verträglichkeit für Mais und Getreide deutlich wirksamer als die vorbekannte Verbindung 2,4-Diamino-6-chlor-5-methylthiopyrimidin.

Die erfindungsgemäßen Wirkstoffe weisen außerdem eine stark mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut des Bodens.

Als Pflanzenschutzmittel können die erfindungsgemäßen Wirkstoffe mit besonders gutem Erfolg zur systemischen Bekämpfung von Reiskrankheiten, wie Pyricularia oryzae eingesetzt werden.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln und/oder festen

0160908

Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z. B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-

Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch als Mischungen mit bekannten Herbziden zur Unkrautbekämpfung Verwendung finden, wobei Fertigformulierung oder Tankmischung möglich ist.

Als Mischpartner bei der Herbizid-Anwendung kommen infrage: Harnstoffe (z.B. Methabenzthiazuron); Diphenylether, Acetanilide (z.B. Alachlor, Metolachlor);

Phenoxyalkancarbonsäuren (z.B. 2,4-D, 2,4-DP, MCPA, MCPP und deren Derivate); Aryloxy- und Hetaryloxy-phenoxypropionsäuren (z.B. 2-[4-(3,5-Dichlor-2-pyridyloxy)-phenoxy]-propionsäure-(trimethylsilylmethyl)-ester; 2-[4-(3,5-Dichlor-2-pyridyloxy)-phenoxy]-propionsäure-(2,2-diethoxy)-ethylester; 2-[4-(3,5-Dichlor-2-pyridyl-oxy)-phenoxy]-propionsäure-(2-benzyloxy)-ethylester); Triazine (z.B. Atrazin, Simazin); Triazinone (z.B. Metribuzin und 4-Amino-6-tert.-butyl-3-ethylthio-1,2,4-triazin-5(4H)-on); Triazindione (z.B. Ametridione).

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen, Tauchen, Vernebeln, Verdampfen, Injizieren, Verschlämmen, Verstreichen, Stäuben, Trockenbeizen, Feuchtbeizen, Naßbeizen, Schlämmbeizen oder Inkrustieren.

Die erfindungsgemäßen Wirkstoffe können bei Herbizid-Anwendung sowohl vor als auch nach dem Auflaufen der Pflanzen appliziert werden. Die Anwendung wird vorzugsweise nach dem Auflaufen der Pflanzen, also im post-emergence-Verfahren, vorgenommen. Sie können auch vor der Saat in den Boden eingearbeitet werden.

Die aufgewandte Wirkstoffmenge kann in größeren Bereichen schwanken. Sie hängt im wesentlichen von der Art des gewünschten Effektes ab. Im allgemeinen liegen die Aufwandmengen bei Herbizid-Anwendungen zwischen 0,01 und 10 kg Wirkstoff pro ha, vorzugsweise zwischen 0,05 und 5 kg pro ha.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens zur Fungizid-Anwendung sind Wirkstoffkonzentrationen von 0,00001 bis 0,1 Gew.-%, vorzugsweise 0,0001 bis 0,02 Gew.-%, am Wirkungsort erforderlich.

Die nachfolgenden Beispiele dienen zur weiteren Erläuterung der Erfindung.

Herstellungsbeispiele

Beispiel 1

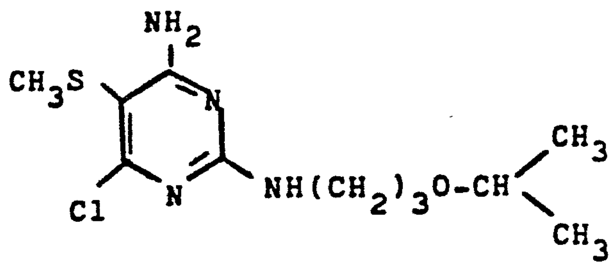

(1)

4-Amino-2-(3-isopropoxypropylamino)-6-chlor-5-methylthiopyrimidin / nach Verfahren (A):

a) 4,6-Dichlor-2-(3-isopropoxypropylamino)-5-methylthiopyrimidin / 1. Stufe:

68,5 g (0,3 Mol) 2,4,6-Trichlor-5-methylthio-pyrimidin werden in 300 ml Toluol gelöst. Hierzu tropft man bei 0°C 30,3 g (0,3 Mol) Triethylamin und 35,1 g (0,3 Mol) Isopropoxypropylamin. Nachdem 1 Stunde bei Raumtemperatur nachgerührt wurde, wird mit 500 ml Toluol verdünnt und die organische Phase mit Wasser gewaschen. Nach Trocknen über Natriumsulfat und Entfernen des Lösungsmittels im Vakuum wird der resultierende Rückstand mit 100 ml Petrolether verrührt, abgesaugt, mit Petrolether nachgewaschen und getrocknet. Man erhält so 25,3 g (27,2 % der Theorie) des obengenannten Pyrimidins, Fp.: 88°C.

b) 4-Amino-2-(3-isopropoxypropylamino)-6-chlor-5-methylthio-pyrimidin / 2. Stufe:

100 g (0,322 Mol) 4,6-Dichlor-2-(3-isopropoxypropylamino)-5-methylthio-pyrimidin werden in 400 ml Methanol gelöst und mit 500 ml Ammoniak im Autoklaven 3 Stunden auf 100°C erhitzt. Der ungelöste Rückstand wird abfiltriert. Nach Einengen der Methanolphase wird der verbleibende Rückstand in Methylenchlorid aufgenommen, mit Wasser gewaschen und die resultierende organische Phase getrocknet. Nach Entfernen des Solvens wird das resultierende Öl mit Petrolether verrührt. Die hierbei erhaltenen Kristalle werden abgesaugt. Man erhält 81 g (87 % der Theorie) des gewünschten Pyrimidins, Fp.: 76°C.

Beispiel 2

4-Amino-2-(3-methoxypropylamino)-6-fluor-5-methylthio-pyrimidin / nach Verfahren (B):

a) 2,6-Difluor-4-amino-5-methylthio-pyrimidin / 1. Stufe:

9 g (0,05 Mol) 2,4,6-Trifluor-5-methylthio-pyrimidin werden in 200 ml Toluol gelöst. Hierzu tropft man bei -30°C 6,8 g (0,1 Mol) 25 %ige wäßrige Ammoniaklösung, rührt viert Stunden bei -30°C und erwärmt auf Raumtemperatur. Nach Zugabe von 300 ml Wasser

wird der entstandene Niederschlag (Produkt A) abgesaugt. Die Toluolphase wird abgetrennt, die resultierende wäßrige Phase zweimal mit 100 ml Toluol extrahiert. Die vereinigten organischen Phasen werden über Natriumsulfat getrocknet und das Lösungsmittel anschließend abgezogen. Es resultiert ein weißer Feststoff (Produkt B).

Produkt A: Ausbeute 1,6 g (18 %), Fp. 245°C
2,6-Difluor-4-amino-5-methylthio-pyrimidin;

Produkt B: Ausbeute 3,5 g (40 %), Fp. 118 bis 121°C
4,6-Difluor-2-amino-5-methylthio-pyrimidin.

Bei größeren Ansätzen (ca. 1 Mol) werden die Isomeren in 75 %iger Gesamtausbeute erhalten.

b) <u>4-Amino-2-(3-methoxypropylamino)-6-fluor-5-methylthio-pyrimidin (Isomerengemisch) / 2. Stufe:</u>

5 g (0,028 Mol) 2,6-Difluor-4-amino-5-methylthiopyrimidin (Produkt A) werden in 100 ml Dioxan bei 80°C mit 5 g (0,056 Mol) 3-Methoxypropylamin drei Stunden unter Rückfluß gekocht. Nach Abkühlen auf Raumtemperatur wird das Reaktionsgemisch in 1,5 l Eiswasser gegeben und mit Methylenchlorid extrahiert. Nach Trocknen über $Na_2SO_4$ und Einengen erhält man 4,2 g (61 % der Theorie) eines Gemisches aus 4-Amino-2-(3-methoxypropylamino)-6-fluor-5-methylthio-pyrimidin und 4-Amino-6-(3-methoxypropylamino)-2-fluor-5-methylthio-pyrimidin, Fp.: 48 bis 64°C.

Auf analogem Wege wurden die in der nachfolgenden Tabelle 1 genannten Pyrimidinderivate der allgemeinen Formel (I)

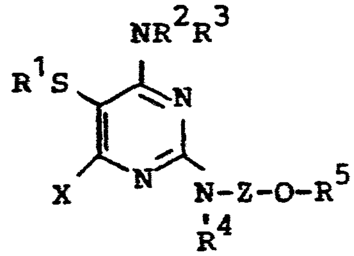

(I)

hergestellt.

Tabelle 1

| Bei-spiel Nr. | X | $R^1$ | $R^2$ | $R^3$ | $R^4$ | Z | $R^5$ | physikal. Konstante |
|---|---|---|---|---|---|---|---|---|
| 3 | Cl | $CH_3$ | H | $CH_3$ | H | $-(CH_2)_3-$ | $i-C_3H_7$ | Fp. 68-71°C |
| 4 | Cl | $CH_3$ | $CH_3$ | $CH_3$ | H | $-(CH_2)_3-$ | $i-C_3H_7$ | $n_D^{20}$ 1.5662 |
| 5 | Cl | $CH_3$ | H | $C_2H_5$ | H | $-(CH_2)_3-$ | $i-C_3H_7$ | Fp. 51-53°C |
| 6 | Cl | $CH_3$ | H | $n-C_3H_7$ | H | $-(CH_2)_3-$ | $i-C_3H_7$ | Fp. 55-58°C |
| 7 | Cl | $CH_3$ | H | $i-C_3H_7$ | H | $-(CH_2)_3-$ | $i-C_3H_7$ | Fp. 55-57°C |
| 8 | Cl | $CH_3$ | H | $n-C_4H_9$ | H | $-(CH_2)_3-$ | $i-C_3H_7$ | Fp. 30°C |
| 9 | Cl | $CH_3$ | H | H | H | $-(CH_2)_3-$ | $CH_3$ | Fp. 116°C |
| 10 | Cl | $CH_3$ | H | $CH_3$ | H | $-(CH_2)_3-$ | $CH_3$ | Fp. 93°C |
| 11 | Cl | $CH_3$ | $CH_3$ | $CH_3$ | H | $-(CH_2)_3-$ | $CH_3$ | Fp. 58°C |
| 12 | Cl | $CH_3$ | H | $C_2H_5$ | H | $-(CH_2)_3-$ | $CH_3$ | Fp. 98°C |
| 13 | Cl | $CH_3$ | H | $n-C_3H_7$ | H | $-(CH_2)_3-$ | $CH_3$ | Fp. 76°C |
| 14 | Cl | $CH_3$ | H | $i-C_3H_7$ | H | $-(CH_2)_3-$ | $CH_3$ | Fp. 68°C |
| 15 | Cl | $CH_3$ | H | $-(CH_2)_3OCH_3$ | H | $-(CH_2)_3-$ | $CH_3$ | Fp. 56°C |
| 16 | F | $CH_3$ | H | H | H | $-(CH_2)_3-$ | $C_2H_5$ | Isomerengemisch: $n_D^{20}$: 1.54 |
| 17 | F | $CH_3$ | H | H | H | $-(CH_2)_2-$ | $CH_3$ | Isomerengemisch: Fp. 86-88°C |

Tabelle 1 (Fortsetzung)

| Beispiel Nr. | X | $R^1$ | $R^2$ | $R^3$ | $R^4$ | Z | $R^5$ | physikal. Konstante |
|---|---|---|---|---|---|---|---|---|
| 18 | F | $CH_3$ | H | $-(CH_2)_3OCH_3$ | H | $-(CH_2)_3-$ | $CH_3$ | Isomerengemisch: $n_D^{20}$: 1,5444 |
| 19 | Cl | $CH_3$ | H | H | H | $-(CH_2)_3-$ | $C_2H_5$ | Fp. 100-104°C |
| 20 | Cl | $CF_3$ | H | H | H | $-(CH_2)_3-$ | $i-C_3H_7$ | Isomerengemisch Fp. 124-142°C |
| 21 | Cl | $CF_3$ | H | H | H | $-CH(CH_3)-(CH_2)_2-$ | $CH_3$ | Fp. 73-75°C |
| 22 | Cl | $CH_3$ | H | H | H | $-(CH_2)_2-$ | $CH_3$ | Fp. 120-122°C |
| 23 | Cl | $CH_3$ | H | $-CH_2-C_4H_9-t$ | H | $-(CH_2)_3-$ | $i-C_3H_7$ | Fp. 90-92°C |

Ausgangsstoffe:

Die als Ausgangsstoffe verwendeten Trihalogenpyrimidine (II-1) und (II-3) und das Dichlorpyrimidinderivat (VIb-1) und deren Herstellung sind bekannt:

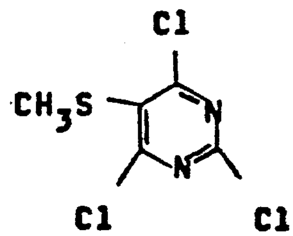

(II-1): bekannt aus FR-A-1.549.494; J. Medicinal Chem. 18, S. 553-8 (1975)

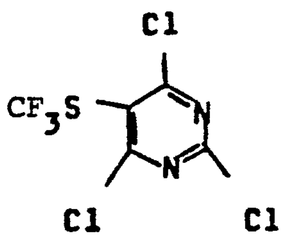

(II-3): bekannt aus M. Lieb, Dissertation, Universität Bochum 1980

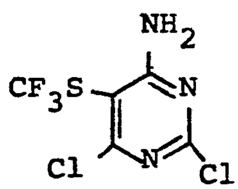

(VIb-1): bekannt aus M. Lieb, Dissertation, Universität Bochum 1980

Das noch nicht vorbeschriebene 2,4,6-Trifluor-5-methylthio-pyrimidin (II-2) kann, ausgehend von (II-1), wie folgt hergestellt werden:

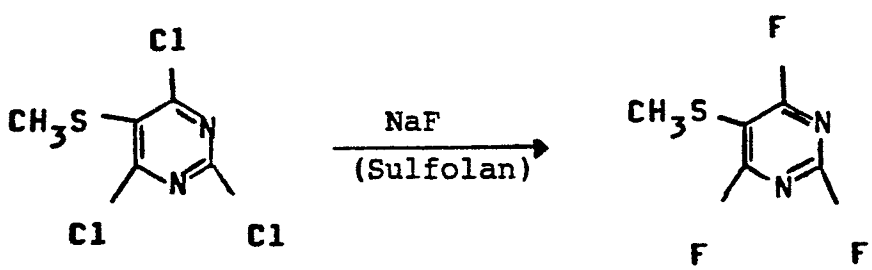


(II-1) (II-2)

In einem 4-Liter-Planschliffgefäß, versehen mit einer Destillationsbrücke, werden 824 g (19,62 Mol) Natriumfluorid und 1430 ml Sulfolan vorgelegt; bei 145°C/20 mbar werden ca. 250 ml Sulfolan abdestilliert. Anschließend gibt man 750 g (3,27 Mol) 2,4,6-Trichlor-5-methylthiopyrimidin (II-1) zu und rührt 4 Stunden bei 200°C. Das Reaktionsgemisch wird danach über die Brücke abdestilliert. Durch Redestillation über eine 50 cm-Füllkörperkolonne erhält man folgende Fraktionen:

a) 483 g 2,4,6-Trifluor-5-methylthio-pyrimidin (II-2) (≙ 82,1 % der Theorie) vom Siedepunkt 72-73°C/20 mbar, $n_D^{20}$: 1,4876;

b) 37 g Nachlauf, Siedebereich 75-120°C/20 mbar; $n_D^{20}$: 1,5211.

Rückstand: 180 g Sulfolan (= Tetramethylensulfon).

Generell werden pro Mol 2,4,6-Trichlor-5-methylthiopyrimidin 6 Mol NaF und pro Mol NaF 60 ml Sulfolan eingesetzt.

Anwendungsbeispiele

In den folgenden Anwendungsbeispielen wurden die nachstehend aufgeführten Verbindungen als Vergleichssubstanzen herangezogen:

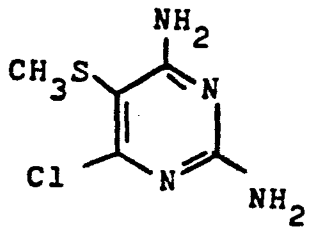

(VS-A)

(bekannt aus EP-A-0 000 681, Beispiel 12);

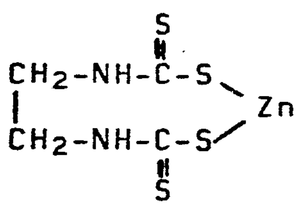

(VS-B)

Zink-ethylen-1,2-bis(dithiocarbamat); common name: Zineb (bekannt z.B. aus R. Wegler "Chemie der Pflanzenschutz- und Schädlingsbekämpfungsmittel", Springer Verlag 1970, Band 2, S. 65 f).

0160908

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

Eine deutliche Überlegenheit in der Wirksamkeit gegenüber dem Stand der Technik zeigen bei diesem Test z.B. die Verbindungen gemäß folgender Herstellungsbeispiele: (1).

0160908

Beispiel B

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß folgender Herstellungsbeispiele:

(13), (14), (16), (19).

0160908

Beispiel C

Pyricularia-Test (Reis) / systemisch

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25°C und einer rel. Luftfeuchtigkeit von 100 % bis zur Auswertung.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß folgender Herstellungsbeispiele:

(14), (16),

Patentansprüche

1. 2,4-Diamino-6-halogen-5-alkylthiopyrimidine der allgemeinen Formel (I)

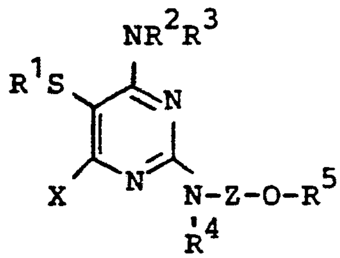

(I)

in welcher

X für Halogen steht,

$R^1$ für gegebenenfalls durch Halogen substituiertes Alkyl steht,

$R^2$ für Wasserstoff oder Alkyl steht,

$R^3$ für Wasserstoff, Alkyl oder Alkoxyalkyl steht,

$R^4$ für Wasserstoff oder Alkyl steht,

Z für eine verzweigte oder unverzweigte Alkylengruppe steht und

$R^5$ für Alkyl steht.

2. Pyrimidinderivate der allgemeinen Formel (I) gemäß Anspruch 1, dadurch gekennzeichnet, daß darin

X für Chlor oder Fluor steht,

$R^1$ für gegebenenfalls durch Chlor und/oder Fluor substituiertes Alkyl mit 1 bis 6 C-Atomen steht,

$R^2$, $R^3$ und $R^4$ jeweils unabhängig voneinander für Wasserstoff oder Alkyl mit 1 bis 6 C-Atomen stehen,

$R^3$ zusätzlich für $(C_1-C_6)$-Alkoxy-$(C_1-C_6)$-alkyl steht,

Z für eine verzweigte oder unverzweigte Alkylengruppe mit 2 bis 10 C-Atomen steht und

$R^5$ für Alkyl mit 1 bis 6 C-Atomen steht.

3. Pyrimidinderivate der allgemeinen Formel (I) gemäß Anspruch 1, dadurch gekennzeichnet, daß darin

X für Chlor oder Fluor steht,

$R^1$ für gegebenenfalls durch Fluor substituiertes Alkyl mit 1 bis 4 C-Atomen steht,

$R^2$, $R^3$ und $R^4$ jeweils unabhängig voneinander für Wasserstoff oder Alkyl mit 1 bis 4 C-Atomen steht,

$R^3$ zusätzlich für $(C_1-C_4)$-Alkoxy-$(C_1-C_4)$-alkyl steht,

Z für eine verzweigte oder unverzweigte Alkylengruppe mit 2 bis 7 C-Atomen steht und

$R^5$ für Alkyl mit 1 bis 4 C-Atomen steht.

4. 4-Amino-2-(3-isopropoxypropylamino)-6-chlor-5-methylthio-pyrimidin der Formel

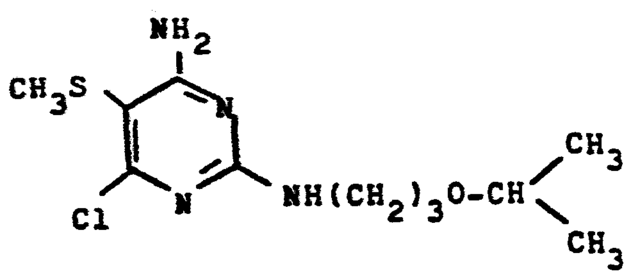

gemäß Anspruch 1.

0160908

5. Verfahren zur Herstellung von 2,4-Diamino-6-halogen-5-alkylthio-pyrimidinen der allgemeinen Formel (I) gemäß Anspruch 1, dadurch gekennzeichnet, daß man Trihalogenpyrimidine der allgemeinen Formel (II)

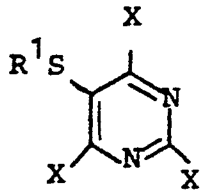

(II)

in welcher

$R^1$ und X die oben angegebene Bedeutung haben, entweder

(A) zunächst mit einem Alkoxyalkylamin der Formel (III)

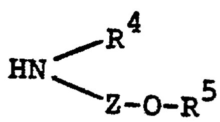

(III)

worin

$R^4$, Z und $R^5$ die oben angegebene Bedeutung haben,

in Gegenwart eines säurebindenden Mittels und gegebenenfalls in Gegenwart eines Verdünnungsmittels zu einem Gemisch der isomeren Pyrimidinderivate der allgemeinen Formeln (IVa) und (IVb)

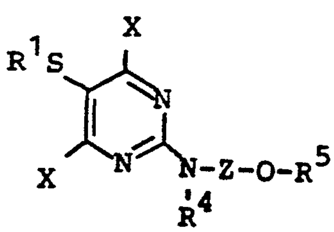

(IVa)

+

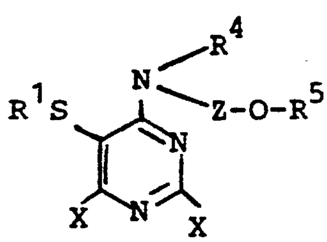

(IVb)

worin

$R^1$, X, $R^4$, Z und $R^5$ die oben angegebene Bedeutung haben,

umsetzt (1. Stufe) und anschließend nach Trennung der beiden Isomeren das Pyrimidinderivat der Formel (IVa) in einem weiteren Reaktionsschritt mit einem Amin der Formel (V)

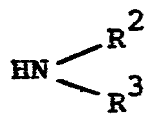

(V)

worin

$R^2$ und $R^3$ die oben angegebene Bedeutung haben,

in Gegenwart eines säurebeindenden Mittels und gegebenenfalls in Gegenwart eines Verdünnungsmittels zum Pyrimidinderivat (I) umsetzt (2. Stufe),

oder

(B) zunächst mit einem Amin der Formel (V) - unter den gleichen Reaktionsbedingungen wie bei Verfahren (A) - zu einem Gemisch der isomeren Pyrimidinderivate der allgemeinen Formeln (VIa) und (VIb)

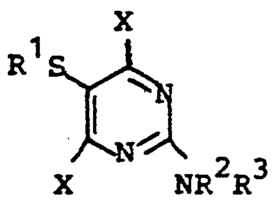

(VIa)

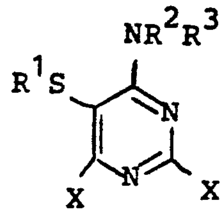

(VIb)

worin

$R^1$, X, $R^2$ und $R^3$ die oben angegebene Bedeutung haben,

umsetzt (1. Stufe) und anschließend nach Trennung der beiden Isomeren das Pyrimidinderivat der Formel (VIb) in einem weiteren Reaktionsschritt mit einem Alkoxyalkylamin der Formel (III) - wiederum unter den gleichen Reaktionsbedingungen wie bei Verfahren (A) - zu einem Gemisch der isomeren Pyrimidinderivate der allgemeinen Formeln (I) und (Ia)

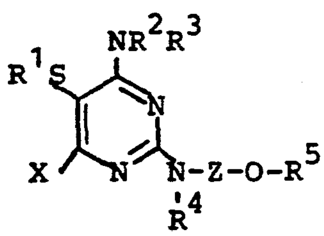

(I)

und

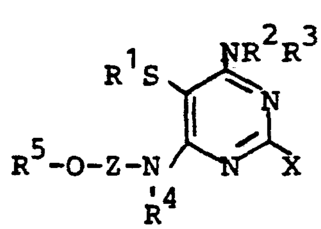

(Ia)

worin

$R^1$, X, $R^2$, $R^3$, $R^4$, Z und $R^5$ die oben angegebene Bedeutung haben,

umsetzt (2. Stufe) und schließlich die gewünschten Isomeren der Formel (I) abtrennt.

6. Herbizide Mittel, gekennzeichnet durch einen Gehalt an mindestens einem 2,4-Diamino-6-halogen-5-alkylthio-pyrimidin der allgemeinen Formel (I) gemäß Anspruch 1.

7. Verwendung von 2,4-Diamino-6-halogen-5-alkylthiopyrimidinen der allgemeinen Formel (I) gemäß Anspruch 1 zur Bekämpfung von unerwünschtem Pflanzenwuchs.

8. Fungizide Mittel, gekennzeichnet durch einen Gehalt an mindestens einem 2,4-Diamino-6-halogen-5-alkylthio-pyrimidin der allgemeinen Formel (I) gemäß Anspruch 1.

9. Verwendung von 2,4-Diamino-6-halogen-5-alkylthiopyrimidinen der allgemeinen Formel (I) gemäß Anspruch 1 zur Bekämpfung von unerwünschten Mikroorganismen.

10. Verfahren zur Herstellung von herbiziden bzw. fungiziden Mitteln, dadurch gekennzeichnet, daß man 2,4-Diamino-6-halogen-5-alkylthio-pyrimidine der allgemeinen Formel (I) gemäß Anspruch 1 mit Streckmitteln und/oder oberflächenaktiven Mitteln vermischt.